# EUROPEAN PATENT SPECIFICATION

(11) **EP 1 044 684 B1**
(45) Date of publication and mention of the grant of the patent: **28.07.2004**
(21) Application number: 00107843.5
(22) Date of filing: 12.04.2000
(51) Int. Cl.: A61K 9/70

(54) **Percutaneously absorbable preparation**
Perkutan absorbierbare Zubereitung
Préparation absorbable par voie percutanée

(30) Priority: 13.04.1999 JP 10590199
(43) Date of publication of application: 18.10.2000
(73) Proprietor: NITTO DENKO CORPORATION, Ibaraki-shi, Osaka 567-8680 (JP)
(72) Inventor: Yamamoto, Keiji, c/o Nitto Denko Corporation, Ibaraki-shi, Osaka 567-8680 (JP); Nakano, Yoshihisa, c/o Nitto Denko Corporation, Ibaraki-shi, Osaka 567-8680 (JP); Hori, Mitsuhiko, c/o Nitto Denko Corporation, Ibaraki-shi, Osaka 567-8680 (JP)
(74) Representative: von Kreisler, Alek, Dipl.-Chem.

(56) References cited:
- US-A- 5 718 914

## Description

The present invention relates to a percutaneously absorptive preparation for transdermally administering a drug except 2-tert-butylamino-1-(2-chloro-4-hydroxyphenyl)ethan-1-ol and a pharmacologically acceptable salt thereof. More particularly, the present invention relates to a practical reservoir type percutaneously absorptive preparation that achieves superior percutaneous absorption when applied to the skin surface, with the aid of a cloth layer formed between a drug reservoir layer-forming film and an adhesive layer.

### BACKGROUND OF THE INVENTION

In recent years, percutaneous administration of various drugs has been actively studied and developed in an attempt to prolong duration of the effect and to reduce side effects. Due to the barrier function of the skin that lowers the skin permeability, however, it is extremely difficult to have the necessary amount of a drug absorbed from the skin upon application to a practical area of the skin.

Generally, percutaneously absorptive preparations mainly consist of monolithic type preparation having two layers of a support and a drug-containing adhesive layer. Because the drug is contained in the adhesive layer, a drug having a lower solubility in the adhesive remains mostly undissolved but exists in a crystalline state. In the course of transfer of the drug into the skin, a drug in a dissolved state can migrate into the skin quickly, but a drug in a crystalline state requires an additional step of dissolution in an adhesive. This step becomes a rate-limiting factor and reduces drug release from the preparation to consequently prevent absorption of a sufficient amount of the drug by the skin. The easiest method to improve this state is to enlarge the area of application or thicken the adhesive layer. Such method is not always effective, because the method in turn causes increased feeling of discomfort during application or longer distance of diffusion for the drug.

When a drug is to be more efficiently administered percutaneously, a method for increasing drug absorption typically includes (1) increasing solubility of the drug in the preparation, which is achieved by either using a specific adhesive or adding a solubilizer, thereby to enhance drug release from the preparation, or (2) improving skin permeability by the addition of a percutaneous absorption promoter.

The above-mentioned monolithic type preparation containing a drug in the adhesive is subject to various limitations when the above method is applied. For example, the solubilizer or percutaneous absorption promoter may not be compatible with the adhesive, or these may be volatilized during heating process. Therefore, a sufficient amount to achieve a desired effect may be too much to be contained in the adhesive. In addition, the adhesive is required to have a higher cohesive force to retain a component, particularly a liquid component, containing a solubilizer and/or a percutaneous absorption promoter. When the adhesive contains a carboxylic group, hydroxyl group or amino group, the adhesive can be crosslinked using a crosslinking agent to achieve higher adhesion. When the above-mentioned liquid component contains a compound having the aforementioned functional group reactive with the crosslinking agent, however, the functional group preferentially reacts with the agent. Consequently, the crosslinking reaction of the adhesive is inhibited, with the result that the liquid component cannot be retained in the adhesive and a desired efficacy cannot be afforded.

Then, the present inventors took note of a reservoir type percutaneously absorptive preparation wherein a drug is sealed in a drug reservoir layer which is located in a drug reservoir layer-forming film or enclosed by a drug reservoir layer-forming film and an impermeable support laminated on the drug reservoir layer-forming film, and an adhesive layer is laminated on the drug reservoir layer-forming film.

The above-mentioned reservoir type preparation comprising a drug reservoir layer containing a drug and a solubilizer shows a weak adhesion between an adhesive layer and a drug reservoir layer-forming film due to a large amount of the liquid component contained in the drug reservoir layer. Consequently, the preparation is associated with a possibility that the drug reservoir layer-forming film may come off during use or when peeling off, leaving the adhesive layer. It frequently happens that the adhesive remains on the skin (i.e., glue remainder) upon peeling off of the preparation. when the drug reservoir layer-forming film is swellable with solubilizer, the preparation itself may deform.

US-A-5,718,914 relates to a device for the transdermal delivery of liposomes. The transdermal device (patch) of US-A-5,718,914 has, as shown in Fig. 1 and Fig. 2, a structure wherein a backing layer, an adhesive layer which bonds the liposome containment member (forming reservoir) thereto, a screen and a cover (e.g., a liner which is peeled away before application to the skin) are sequentially laminated. The screen is located between the liposome containment member and the cover.

It is therefore an object of the present invention to provide a percutaneously absorptive preparation superior in percutaneous absorption of the drug, which is capable of inhibiting a glue remainder on the skin upon peeling off and inhibiting swelling or deformation of a drug reservoir layer-forming film by a solvent of a drug contained in a drug reservoir layer.

### SUMMARY OF THE INVENTION

The present invention relates to a percutaneously absorptive preparation comprising a drug reservoir layer, a drug reservoir layer-forming film, a cloth layer, an adhesive layer and optionally an impermeable support, wherein the drug reservoir layer is enclosed in the drug reservoir layer-forming film or enclosed by the drug reservoir layer-forming film and the support formed on the drug reservoir layer-forming film, the drug reservoir layer contains a drug except 2-tert-butylamino-1-(2-chloro-4-hydroxyphenyl)ethan-1-ol and a pharmacologically acceptable salt thereof, characterized in that the cloth layer is located between the adhesive layer and the drug reservoir layer-forming film.

Preferred embodiments become apparent from the dependent claims.

The present invention is characterized by a cloth layer formed between a drug reservoir layer-forming film and an adhesive layer. The characteristic layer of the present invention provides beneficial effects of the elimination of a glue remainder and deformation of the film, and superior percutaneous absorption even by application to a small area of the skin.

The percutaneously absorptive preparation of the present invention characteristically has a structure wherein a drug (except 2-tert-butylamino-1-(2-chloro-4-hydroxyphenyl)ethan-1-ol and a pharmacologically acceptable salt thereof) is sealed in a drug reservoir layer which is located in a drug reservoir layer-forming film or which is enclosed by a drug reservoir layer-forming film and an impermeable support formed on the drug reservoir layer-forming film. An adhesive layer is further laminated on the drug reservoir layer-forming film, and a cloth layer is formed between the adhesive layer and the drug reservoir layer-forming film. The drug reservoir layer preferably contains, besides the drug, a solubilizer (preferably C₁-C₄ alcohol) and/or a percutaneous absorption promoter (preferably at least one kind from ester of C₁₂-C₁₈ fatty acid, diester of C₆-C₁₀ dicarboxylic acid and glycerol ester of C₈-C₁₀ fatty acid). The drug reservoir layer-forming film is preferably made from an ethylene-vinyl acetate copolymer containing vinyl acetate by 5 - 40 wt%, and a pH adjusting agent is preferably contained in the drug reservoir layer and/or the adhesive layer.

As mentioned above, the preparation of the present invention comprises a cloth layer between a drug reservoir layer-forming film and an adhesive layer of the conventional reservoir type preparations. The cloth layer functions as a backing of the drug reservoir layer-forming film. This has an effect that the deformation of the preparation due to the swelling of the drug reservoir layer-forming film can be inhibited. The cloth layer shows superior close adhesion to the adhesive layer. As a result, the adhesive layer stays closely adhered to the cloth layer, whereby the adhesive remains less frequently on the skin.

### BRIEF DESCRIPTION OF THE DRAWINGS

Fig. 1 shows a schematic view of the cross section of one embodiment of the percutaneously absorptive preparation of the present invention.

Fig. 2 shows a schematic view of the cross section of another embodiment of the percutaneously absorptive preparation of the present invention.

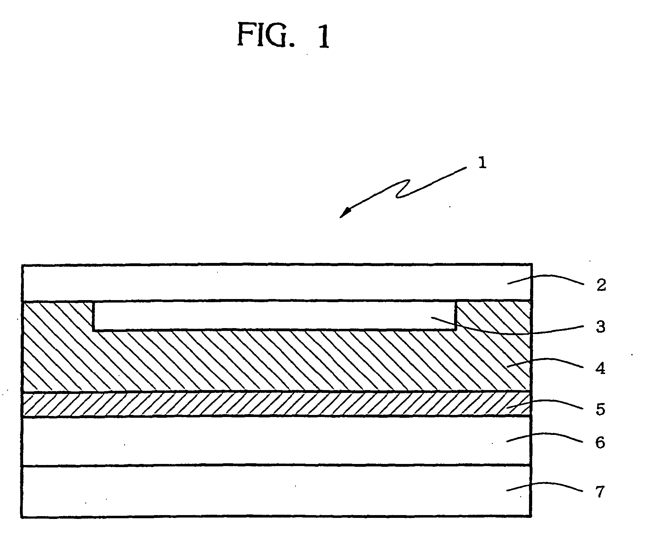

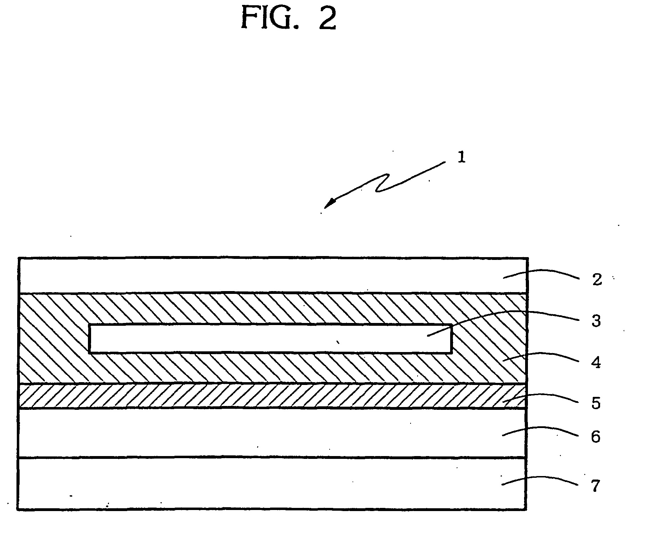

In Figs. 1 and 2, 1 is a percutaneously absorptive preparation, 2 is a support, 3 is a drug reservoir layer, 4 is a drug reservoir layer-forming film, 5 is a cloth layer, 6 is an adhesive layer, and 7 is a release liner.

### DETAILED DESCRIPTION OF THE INVENTION

As shown in Figs. 1 and 2, the drug reservoir layer 3 may be enclosed in the drug reservoir layer-forming film 4 itself, or enclosed by a drug reservoir layer-forming film 4 and an impermeable support 2 formed on the drug reservoir layer-forming film 4.

The drug reservoir layer-forming film may be a single layer of a heat sealable polymer film of, for example, polyethylene, polypropylene, ethylene-vinyl acetate copolymer, ethylene-vinyl alcohol copolymer and the like, or a laminate of these.

Because of the high permeability to polar solvents, an ethylene-vinyl acetate copolymer is preferable, more preferably that having a vinyl acetate content of 5 wt% - 40 wt%. When the vinyl acetate content is less than 5 wt%, the solubilizer shows lower film permeability, with the consequence of insufficient transfer of the drug to an adhesive layer (layer 6 in Figs. 1, 2). When it exceeds 40 wt%, the film itself has an insufficient strength.

The drug reservoir layer-forming film preferably has a thickness of 10 - 100 µm, more preferably 20 - 50 µm.

The impermeable support laminated on the drug reservoir layer-forming film is impermeable to a reservoir composition (to be also referred to as a drug-containing liquid composition) in the drug reservoir layer. It is preferably heat sealable with the drug reservoir layer-forming film to form the periphery of the preparation. For example, polyethylene, polypropylene, ethylene-vinyl acetate copolymer, ethylene-vinyl alcohol copolymer and the like can be used. To prevent loss of the content of the drug reservoir layer through the support of the preparation, a film (e.g., polyester, a metal foil of aluminum and the like) impermeable to the drug-containing liquid composition in the drug reservoir layer, which contains a drug, a percutaneous absorption promoter, a solvent and the like, is preferably laminated.

The impermeable support has a thickness of generally 5 - 500 µm, preferably 5 - 200 µm. The support is preferably subjected to corona discharge treatment, plasma treatment, oxidation treatment and the like of the surface, on which a drug reservoir layer-forming film is formed, to achieve higher adhesion of the support to the drug reservoir layer-forming film and an anchor effect.

The cloth layer (layer 5 in Figs. 1, 2) formed between the drug reservoir layer-forming film and the adhesive layer is heat melt adhered or press adhered to the forming film. The cloth is free of any particular limitation as long as it is porous, adheres well to the adhesive layer and is capable of heat melt adhesion or press adhesion to the drug reservoir layer-forming film. Examples thereof include woven fabric, nonwoven fabric and the like. The cloth layer is made from polyester, polypropylene, rayon, nylon, cellulose and the like.

The cloth layer may be laminated on a porous film. The cloth layer in the present invention encompasses such a laminate. The porous film preferably is one usable as the above-mentioned drug reservoir layer-forming film, or one having air permeability (Gurley method) of not more than 9000 sec/100 cc. The laminate is used in such a manner that the adhesive layer is in contact with the cloth.

The cloth layer has a superior anchor effect that allows for strong adhesion to the adhesive layer. This has a consequence that, even if a solubilizer causes marked plasticization of adhesive, the drug reservoir layer-forming film does not come off during application or upon peeling off, leaving the adhesive layer, and a glue remainder on the skin surface is inhibited upon peeling off. The cloth layer prevents swelling and deformation of the drug reservoir layer-forming film containing a large amount of the liquid component. Even if the drug reservoir layer-forming film is swellable with the solubilizer to be used, the presence of a cloth layer prevents deformation of the preparation of the present invention.

The drug reservoir layer completely seals a drug other than 2-tert-butylamino-1-(2-chloro-4-hydroxyphenyl)ethan-1-ol and pharmacologically acceptable salts thereof. This drug may be in the form of a pharmacologically acceptable salt or a prodrug.

The drug to be sealed in the drug reservoir layer may be any and is exemplified by corticosteroides, analgesic antiphlogistic, somnifacient sedative agent, tranquilizer, antihypertensive, hypotensive diuretic, antibiotic, anesthetic, antimicrobial, antifungal agent, vitamin agent, coronary vasodilator, antihistamine, cough medicine, sex hormone agent, antidepressant, cerebral circulation improver, antiemetic, antitumor agent, biological drug and the like. In particular, the drug preferably has a solubility of not less than 1 wt% in a solubilizer to be mentioned later.

The composition to be sealed in the drug reservoir layer has a drug content that is subject to change depending on the kind of the drug and the like. It is generally preferably 0.5 - 40 wt%. When it is less than 0.5 wt%, the therapeutic effect is insufficient and the content exceeding 40 wt% is unpreferable from the aspect of drug utilization.

The drug reservoir layer preferably additionally seals a solubilizer and/or a percutaneous absorption promoter.

The mixing ratio of the solubilizer and the drug can vary depending on the object of the preparation, the drug to be sealed and the like. The proportion of the drug is generally preferably 0.1 - 200 parts by weight, more preferably 1 - 100 parts by weight, per 100 parts by weight of the solubilizer.

The mixing ratio of the percutaneous absorption promoter and the drug can vary depending on the object of the preparation, the drug to be sealed and the like. The proportion of the drug is generally preferably 0.5 - 2000 parts by weight, more preferably 1 - 1000 parts by weight, per 100 parts by weight of the percutaneous absorption promoter.

When the drug reservoir layer seals a solubilizer and a percutaneous absorption promoter, the mixing ratio of the two is preferably 3 - 200 parts of the percutaneous absorption promoter by weight per 100 parts by weight of the solubilizer. When it is less than 3 parts by weight, the adhesive cannot contain a sufficient amount of the percutaneous absorption promoter, which in turn results in a failure to achieve a desired promoting effect. When it exceeds 200 parts by weight, the relative amount of the solubilizer in the drug reservoir layer decreases, undesirably lowering the solubility of the drug.

The solubilizer is added to increase solubility of the drug. The dissolved drug permeates the skin preferably with the aid of the percutaneous absorption promoter. Therefore, the solubilizer preferably dissolves the drug and is miscible with the percutaneous absorption promoter to be mentioned later. Examples of the solubilizer include lower alcohol such as ethyl alcohol and the like, polyhydric alcohol such as ethylene glycol, glycerol and the like, dimethyl sulfoxide, dimethylacetamide, dimethylformamide, N-methyl-2-pyrrolidone, dimethyllaurylamide, dodecylpyrrolidone, isosorbitol, liquid paraffin, various liquid surfactants, mineral oil, lanolin, ethyl acetate, crotamiton, water and the like. Preferable solubilizer may be lower alcohol having 1 to 4 carbon atoms. The alcohol having 1 to 4 carbon atoms is exemplified by methyl alcohol, ethyl alcohol, n-propyl alcohol, isopropyl alcohol, n-butyl alcohol, isobutyl alcohol and tert-butyl alcohol.

The percutaneous absorption promoter may be glycol such as ethylene glycol and the like, fats and oils such as olive oil, squalene and the like, polar solvent such as dimethyl sulfoxide, dimethylformamide and the like, surfactant such as sodium lauryl sulfate, sorbitan fatty acid ester and the like, long chain fatty acid such as oleic acid and the like, and the like. From the aspect of increased percutaneous absorption of the drug, compatibility with the above-mentioned solubilizer and the like, at least one kind selected from esters of fatty acid having 12 to 18 carbon atoms, diesters of dicarboxylic acid having 6 to 10 carbon atoms and glycerol esters of fatty acid having 8 to 10 carbon atoms is preferably used.

As the fatty acid ester, an alkyl ester of aliphatic monocarboxylic acid having 12 to 18 carbon atoms such as lauric acid, myristic acid, palmitic acid, stearic acid, oleic acid and the like, and alcohol having 1 to 10 carbon atoms such as methyl alcohol, ethyl alcohol, n-propyl alcohol, isopropyl alcohol, n-butyl alcohol, isobutyl alcohol, pentyl alcohol, hexyl alcohol, heptyl alcohol, octyl alcohol, nonyl alcohol, isononyl alcohol and the like may be used.

The diester of dicarboxylic acid is preferably dialkyl esters of aliphatic dicarboxylic acid having 6 to 10 carbon atoms, such as diester of adipic acid or sebacic acid and alcohol having 1 to 10 carbon atoms.

The glycerol ester of fatty acid is exemplified by glycerol ester of fatty acid having 8 to 10 carbon atoms, such as glyceryl monocaprylate, glyceryl monocaprate, tricaprylin and the like.

For an improved manipulability of the composition to be sealed in the drug reservoir layer during production, glycerol, propylene glycol, polyethylene glycol, poly(vinylpyrrolidone), hydroxypropylcellulose and the like may be added to increase viscosity.

When the drug is in the form of a salt, a pH adjusting agent is preferably contained in the drug reservoir layer and/or adhesive layer. By adding a basic pH adjusting agent, such as sodium hydroxide, triethanolamine, triisopropanolamine, diisopropanolamine, monoisopropanolamine, sodium caprylate, potassium hydroxide and the like, or an acidic pH adjusting agent, such as citric acid, lactic acid, glyconic acid, succinic acid, tartaric acid, lactic acid, maleic acid and the like, the drug in the form of a salt can be liberated. As a result, the drug can be converted to a drug in the form of a base or an acid capable of higher percutaneous absorption. The content of the pH adjusting agent is such an amount as is necessary for neutralizing the acid or base attached to the drug in the form of a base or an acid.

When the content of the pH adjusting agent is less than this level, a base or an acid is generated in a less amount. The percutaneous absorption may become somewhat inferior, but the stability of the drug in the preparation becomes superior. When the content is higher, the adhesive layer becomes basic or acidic, and may induce irritation to the skin when applied.

The amount of the composition to be sealed in the drug reservoir layer is preferably not more than 50 µL/cm². When it exceeds 50 µL/cm², it may happen that the composition that cannot be retained in the drug reservoir layer or the composition that was transferred from the drug reservoir layer into the adhesive layer cannot be retained in the adhesive. It has a consequence of separation of the adhesive component (blooming) or marked plasticization of the adhesive, which in turn may cause a glue remainder on the skin surface upon peeling off.

The adhesive layer and/or drug reservoir layer may contain, where necessary, in addition to the above-mentioned pH adjusting agent and percutaneous absorption promoter, various known additives such as solubilizer, stabilizer represented by antioxidant (e.g., ascorbic acid, tocopherol, dibutylhydroxytoluene and the like), plasticizer (e.g., glycerol, propylene glycol, polyethylene glycol and the like), filler (e.g., titanium oxide, zinc oxide, water-containing silicon dioxide and the like), and the like.

The adhesive layer of the percutaneously absorptive preparation may cover the entire surface of a cloth layer, or may cover only an appropriate width on the periphery of the cloth layer forming a frame-like shape.

The polymer to be used for the adhesive layer is a pressure sensitive adhesive polymer that shows adhesive property at normal temperature. Preferably, one compatible with the aforementioned solubilizer-percutaneous absorption promoter mixture is used. Preferable examples of such adhesive include acrylic adhesive and rubber adhesive.

Examples of the acrylic adhesive include homopolymer such as alkyl acrylate and alkyl methacrylate, and copolymers thereof. The alkyl of the alkyl acrylate and alkyl methacrylate means linear or branched chain alkyl having 1 to 18 carbon atoms, which is specifically methyl, ethyl, propyl, butyl, pentyl, hexyl, heptyl, octyl, isooctyl, 2-ethylhexyl, nonyl, isononyl, decyl, undecyl, dodecyl, tridecyl, tetradecyl, pentadecyl, hexadecyl, heptadecyl, octadecyl and the like. Preferred is linear or branched chain alkyl having 4 to 12 carbon atoms. The acrylic adhesive to be used in the present invention may be a copolymer of the aforementioned alkyl acrylate and/or alkyl methacrylate with one or more kinds of the monomers mentioned below.

The monomer is exemplified by those having carboxylic group (e.g., acrylic acid, itaconic acid, maleic acid, maleic anhydride), those having sulfonic acid group (e.g., styrenesulfonic acid, allylsulfonic acid, sulfopropyl acrylate, acryloyloxynaphthalenesulfonic acid, acrylamidomethylpropanesulfonic acid), hydroxy lower alkyl acrylate (e.g., hydroxymethyl acrylate, hydroxyethyl acrylate, hydroxypropyl acrylate, hydroxybutyl acrylate), acrylamide, acrylamide derivative (e.g., dimethylacrylamide, N-butylacrylamide, N-methylolacrylamide, N-methylolpropaneacrylamide), aminoalkyl acrylate (e.g., aminoethyl acrylate), alkylaminoalkyl acrylate (e.g., dimethylaminoethyl acrylate, tert-butylaminoethyl acrylate), alkoxyalkyl acrylate (e.g., methoxyethyl acrylate, ethoxyethyl acrylate), tetrahydrofurfuryl acrylate, ester of acrylic acid and methoxydiethylene glycol, ester of acrylic acid and methoxypolyethylene glycol, ester of acrylic acid and methoxypolypropylene glycol, hydroxy lower alkyl methacrylate (e.g., hydroxymethyl methacrylate, hydroxyethyl methacrylate, hydroxypropyl methacrylate, hydroxybutyl methacrylate), methacrylamide, methacrylamide derivative (e.g., dimethylmethacrylamide, N-butylmethacrylamide, N-methylolmethacrylamide, N-methylolpropane methacrylamide), aminoalkyl methacrylate (e.g., aminoethyl methacrylate), alkylaminoalkyl methacrylate (e.g., dimethylaminoethyl methacrylate, tert-butyl aminoethyl methacrylate), alkoxyalkyl methacrylate (e.g., methoxyethyl methacrylate, ethoxyethyl methacrylate), tetrahydrofurfuryl methacrylate, ester of methacrylic acid and methoxydiethylene glycol, ester of methacrylic acid and methoxypolyethylene glycol, ester of methacrylic acid and methoxypolypropylene glycol, methacrylonitrile, acrylonitrile, vinyl acetate, vinyl propionate, vinylpyrrolidone, methylvinylpyrrolidone, vinyl pyridine, vinylpiperidone, vinylpyrimidine, vinylpiperazine, vinylpyrazine, vinylpyrrole, vinylimidazole, vinylcaprolactam, vinyloxazole, vinylmorpholine, styrene and the like.

When a copolymer of alkyl acrylate and/or alkyl methacrylate with the above-mentioned monomers is used as the acrylic adhesive, 30 - 99.5 wt%, preferably 50 - 90 wt% of the alkyl acrylate and/or alkyl methacrylate and 0.5 - 70 wt%, preferably 10 - 50 wt%, of the above-mentioned monomer are preferably copolymerized.

When the above-mentioned acrylic adhesive has a crosslinked structure, sufficient cohesive force can be achieved even when the solubilizer or percutaneous absorption promoter plasticizes the adhesive layer noticeably. A crosslinking treatment can be applied only when the acrylic adhesive comprises a monomer having a carboxylic group or hydroxyl group as an element. Examples of the crosslinking agent include metal alcoholate of titanium or aluminum, metal chelate compound, multifunctional isocyanate (particularly trifunctional isocyanate) and the like.

These crosslinking agents are generally added in a proportion of 0.05 - 5 parts by weight per 100 parts by weight of acrylic polymer.

The rubber adhesive preferably contains a high molecular weight rubber polymer having a viscosity-average molecular weight of 300,000 - 2,500,000. The high molecular weight rubber polymer is an essential element to afford an appropriate cohesive force to the rubber adhesive. It is preferably contained in the rubber adhesive in a proportion of not less than 10 wt%, more preferably not less than 20 wt%. Examples of the high molecular weight rubber polymer include polyisobutylene, polyisoprene, polybutadiene, styrene-isoprene-styrene block copolymer, styrene-butadiene-styrene block copolymer and the like, which may be used alone or in combination.

The rubber adhesive may contain tackifier such as rosin resin, polyterpene resin, chroman-indene resin, petroleum resin, terpene-phenol resin, xylene resin, alicyclic saturated hydrocarbon resin and the like, to impart adequate adhesive property.

Preferable rubber adhesive contains a high molecular weight polyisobutylene having a viscosity-average molecular weight of 300,000 - 2,500,000, and further, a medium molecular weight polyisobutylene having a viscosity-average molecular weight of 10,000 - 200,000 and/or a low molecular weight polyisobutylene or polybutene having a viscosity average molecular weight of 500 - 4,000. The rubber adhesive preferably contains the high molecular weight polyisobutylene in a proportion of 10 - 80 wt%, preferably 20 - 70 wt%, the medium molecular weight polyisobutylene in a proportion of 0 - 90 wt%, preferably 10 - 80 wt%, and the low molecular weight polyisobutylene or polybutene in a proportion of 0 - 80 wt%, preferably 10 - 60 wt%. In the present invention, the viscosity-average molecular weight is calculated from the viscosity equation of Flory.

The adhesive layer preferably has a thickness of 10 - 100 µm, which secures sufficient adhesion to the skin for a long time.

The adhesive layer may contain the above-mentioned percutaneous absorption promoter. The percutaneous absorption promoter is contained in a proportion of not more than 100 parts by weight per 100 parts by weight of the adhesive polymer. When the content exceeds 100 parts by weight, it may not be retained in the adhesive. As a result, separation (blooming) of the percutaneous absorption promoter from the adhesive component may occur on the surface of the adhesive, which degrades adhesion to the skin.

In the percutaneously absorptive preparation of the present invention, a release liner (reference symbol 7 in Figs. 1, 2) desirably covers and protects the exposed surface of the adhesive layer. The release liner is subject to no particular limitation as long as it can be peeled off easily from the adhesive layer immediately before use. For example, a film made from polyester, poly(vinyl chloride), poly(vinylidene chloride), poly(ethylene terephthalate) and the like, paper such as fine paper, glassine paper and the like, or a laminate film of polyolefin and fine paper, glassine paper and the like may be used after peeling off treatment involving application of silicone resin, fluororesin and the like onto the surface to be in contact with the adhesive layer. The release liner has a thickness of generally 10 - 200 µm, preferably 50 - 100 µm.

The cross sections of the percutaneously absorptive preparation of the present invention are shown in Figs. 1 and 2.

The production method of the percutaneously absorptive preparation of the present invention is not particularly limited. For example, an adhesive is applied onto a release liner and dried, a cloth is laminated thereon and the laminate is subjected to heat melt adhesion with a drug reservoir layer-forming film. The impermeable support is heat sealed at a suitable sealing width, leaving a suitable inner diameter of the drug reservoir layer, except a drug reservoir layer solution inlet, and cut along the periphery of the heat sealed part. A drug reservoir layer solution containing the drug is injected thereinto and the inlet is completely closed by heat sealing, whereby the percutaneously absorptive preparation of the present invention is obtained.

When the drug reservoir layer is formed in the drug reservoir layer-forming film itself, a drug reservoir layer-forming film is laminated in advance on either side of the impermeable support. Separately, an adhesive layer, a cloth and a drug reservoir layer-forming film are successively laminated. The two drug reservoir layer-forming films of the resulting laminates are heat sealed leaving the inlet. In the same manner as above, a drug reservoir layer solution is injected from the inlet and the inlet is completely heat sealed to give the percutaneously absorptive preparation of the present invention.

The present invention is explained in more detail by referring to illustrative examples. The compositions of the examples are shown in Table 1 and Table 2. As used herein, "parts" and "%" mean "parts by weight" and "wt%", respectively.

### Preparation of adhesive solution A

2-Ethylhexyl acrylate (95 parts) and acrylic acid (5 parts) were polymerized in ethyl acetate under an inert gas atmosphere to give an acrylic adhesive solution A.

### Preparation of adhesive solution B

High molecular weight polyisobutylene (50 parts, viscosity-average molecular weight 2,100,000, VISTANEX MML-140, manufactured by Exxon Chemicals Japan LTD.), medium molecular weight polyisobutylene (30 parts, viscosity-average molecular weight 60,000, HIMOL 6H, manufactured by NIPPON PETROCHEMICALS CO., LTD.) and alicyclic petroleum resin (20 parts, softening point 100°C, Arkon P-100, manufactured by ARAKAWA CHEMICAL INDUSTRIES LTD.) were dissolved in hexane to give a rubber adhesive solution B.

### Example 1

To the adhesive solution A was added and mixed a polyisocyanate compound (CORONATE HL, manufactured by NIPPON POLYURETHANE INDUSTRY CO., LTD., solid content 0.15% in adhesive). The mixture was applied onto a release liner (75 µm thick polyester film after peeling off treatment) to a thickness after drying of 40 µm and dried. A polyester nonwoven fabric (basic weight 8 g/m²) was laminated thereon and a 50 µm thick ethylene - vinyl acetate copolymer film (vinyl acetate content 14%) was heat melt adhered. A support (a laminate of aluminum-deposited polyester film and polyethylene) was heat sealed therewith leaving a drug reservoir layer solution inlet in the size of inner diameter 20 mm, seal width 4 mm, and cut along the periphery of the heat sealed part.

A drug reservoir layer solution (50 µL, containing an ethyl alcohol-isopropyl myristate mixture (mixing ratio 7:3) and ketoprofen at a concentration of 10%) was injected from the inlet and the inlet was completely heat sealed to give the percutaneously absorptive preparation of the present invention.

### Example 2

In the same manner as in Example 1 except that the drug reservoir layer solution contained an ethyl alcohol-isopropyl myristate-glyceryl monocaprylate mixture (mixing ratio 7:2:1) and ketoprofen at a concentration of 10%, the percutaneously absorptive preparation of the present invention was obtained.

### Example 3

In the same manner as in Example 1 except that the drug reservoir layer solution contained an ethyl alcohol-diethyl sebacate mixture (mixing ratio 7:3) and ketoprofen at a concentration of 10%, the percutaneously absorptive preparation of the present invention was obtained.

### Example 4

In the same manner as in Example 1 except that isopropyl myristate was added to the adhesive solution B in a proportion of 40% and the drug reservoir layer solution contained an isopropyl alcohol-isopropyl myristate mixture (mixing ratio 6:4) and metoprolol at a concentration of 10%, the percutaneously absorptive preparation of the present invention was obtained.

### Example 5

In the same manner as in Example 4 except that the ethylene-vinyl acetate copolymer had a vinyl acetate content of 5% and the drug reservoir layer solution contained an ethyl alcohol-isopropyl myristate mixture (mixing ratio 6:4) and metoprolol at a concentration of 10%, the percutaneously absorptive preparation of the present invention was obtained.

### Example 6

In the same manner as in Example 4 except that the ethylene-vinyl acetate copolymer had a vinyl acetate content of 33% and the drug reservoir layer solution contained an ethyl alcohol-isopropyl myristate mixture (mixing ratio 6:4) and metoprolol at a concentration of 10%, the percutaneously absorptive preparation of the present invention was obtained.

### Example 7

In the same manner as in Example 4 except that the drug reservoir layer solution contained an ethyl alcohol-isopropyl myristate mixture (mixing ratio 6:4) and metoprolol tartrate (concentration 10%) and triethanolamine (concentration 7%), the percutaneously absorptive preparation of the present invention was obtained.

### Comparative Example 1 (reservoir type)

In the same manner as in Example 1 except that the polyester nonwoven fabric was not laminated between the adhesive layer and the drug reservoir layer-forming film, the percutaneously absorptive preparation of the present invention was obtained.

### Comparative Example 2 (monolithic type)

Ketoprofen and isopropyl myristate were added to the adhesive solution A to a content in a plaster of 10% and 40%, respectively, and mixed. The mixture was applied onto a release liner to a thickness after drying of 40 µm and dried. A support (12 µm-thick polyester film) was attached thereto to give a percutaneously absorptive preparation.

### Experimental Example

The amount of the drug that permeated the skin was measured using the percutaneously absorptive preparations of Examples 1-7 and Comparative Examples 1, 2, and a rat abdominal skin that had been removed and depilated.

### Measurement method

The rat skin was attached to a glass diffusion cell such that the surface of the skin was in contact with the above-mentioned percutaneously absorptive preparation and the back of the skin was in contact with physiological saline. The drug that permeated into physiological saline was measured by high performance liquid chromatography. The results are shown in Tables 1, 2 and 3.

**Table 1**

| | | Drug reservoir layer | |
|---|---|---|---|
| | | Solubilizer/percutaneous absorption promoter | Drug, pH adjusting agent |
| Example | 1 | ethyl alcohol/IPM=7/3 | Ketoprofen 10% |
| | 2 | ethyl alcohol/IPM/GMC=7/2/1 | Ketoprofen 10% |
| | 3 | ethyl alcohol/DES=7/3 | Ketoprofen 10% |
| | 4 | isopropyl alcohol/IPM=6/4 | Metoprolol 10% |
| | 5 | ethyl alcohol/IPM=6/4 | Metoprolol 10% |
| | 6 | ethyl alcohol/IPM=6/4 | Metoprolol 10% |
| | 7 | ethyl alcohol/IPM=6/4 | Metoprolol tartrate 10%, triethanolamine 7% |
| Comp. Ex. | 1 | ethyl alcohol/IPM=7/3 | Ketoprofen 10% |
| | 2 | * | Ketoprofen 10% |

| | | | |
|---|---|---|---|
| * Matrix type preparation containing a drug and a percutaneous absorption promoter in the adhesive layer. IPM: isopropyl myristate GMC: glyceryl monocaprylate DES: diethyl sebacate | | | |

**Table 2**

| | | Vinyl acetate content of drug reservoir layer-forming film | Cloth | Adhesive layer | |
|---|---|---|---|---|---|
| | | | | Adhesive | Percutaneous absorption promoter |
| Example | 1 | 14% | polyester unwoven fabric | A | none |
| | 2 | 14% | polyester unwoven fabric | A | none |
| | 3 | 14% | polyester unwoven fabric | A | none |
| | 4 | 14% | polyester unwoven fabric | B | IPM40% |
| | 5 | 5% | polyester unwoven fabric | B | IPM40% |
| | 6 | 33% | polyester unwoven fabric | B | IPM40% |
| | 7 | 14% | polyester unwoven fabric | B | IPM40% |
| Comp. Ex. | 1 | 14% | none | A | none |
| | 2 | - | - | A | IPM40% |
| IPM: isopropyl myristate GMC: glyceryl monocaprylate DES: diethyl sebacate | | | | | |

**Table 3**

| | | Accumulated skin permeation (µg/cm²/24h) |
|---|---|---|
| Example | 1 | 1398 |
| | 2 | 1089 |
| | 3 | 1205 |
| | 4 | 1297 |
| | 5 | 908 |
| | 6 | 1602 |
| | 7 | 1088 |
| Comparative Example | 1 | 1174 |
| | 2 | 420 |

The preparation of Comparative Example 1 showed markedly greater deformation of the preparation such as wrinkles and ripples, as compared to the preparation of the present invention. When peeled off from the skin, the drug reservoir layer-forming film was separated from the adhesive layer and the adhesive remained on the skin.

The percutaneously absorptive preparations of Examples 1-3 showed fine skin permeability as compared to the preparation of Comparative Example 2.

The percutaneously absorptive preparation of the present invention including a cloth layer between the drug reservoir layer-forming film and the adhesive layer showed strong adhesion of the drug reservoir layer-forming film to the adhesive layer, thereby inhibiting separation of the drug reservoir layer-forming film from the adhesive layer, and glue remainder on the skin after peeling off. The presence of the cloth layer inhibits the deformation of the preparation itself even when the drug reservoir layer-forming film is swellable with a solubilizer.

In addition, the preparation of the present invention is superior in the percutaneous absorption of a drug.

## Claims

1. A percutaneously absorptive preparation (1) comprising a drug reservoir layer (3), a drug reservoir layer-forming film (4), a cloth layer (5), an adhesive layer (6) and optionally an impermeable support (2), wherein the drug reservoir layer (3) is enclosed in the drug reservoir layer-forming film or enclosed by the drug reservoir layer-forming film (4) and the support formed on the drug reservoir layer-forming film (4), the drug reservoir layer (3) contains a drug except 2-tert-butylamino-1-(2-chloro-4-hydroxyphenyl)ethan-1-ol and a pharmacologically acceptable salt thereof, **characterized in that** the cloth layer (5) is located between the adhesive layer (6) and the drug reservoir layer-forming film (4).

2. The percutaneously absorptive preparation (1) of claim 1, wherein the drug reservoir layer (3) further seals a solubilizer or a percutaneous absorption promoter, or a solubilizer and a percutaneous absorption promoter.

3. The percutaneously absorptive preparation (1) of claim 2, wherein the solubilizer is alcohol having 1 to 4 carbon atoms.

4. The percutaneously absorptive preparation (1) of claim 2, wherein the percutaneous absorption promoter is at least one member selected from the group consisting of an ester of fatty acid having 12 to 18 carbon atoms, a diester of dicarboxylic acid having 6 to 10 carbon atoms and a glycerol ester of fatty acid having 8 to 10 carbon atoms.

5. The percutaneously absorptive preparation of claim 1, wherein the drug reservoir layer-forming film (4) is made from an ethylene-vinyl acetate copolymer having a vinyl acetate content of 5-40 wt%.

6. The percutaneously absorptive preparation of claim 1, further comprising a pH adjusting agent in the drug reservoir layer (3) or the adhesive layer (6) or the drug reservoir layer (3) and the adhesive layer (6).

## Patentansprüche

1. Perkutan resorbierbares Präparat (1), das eine Wirkstoffreservoirschicht (3), einen die Wirkstoffreservoirschicht bildenden Film (4), eine Textilschicht (5), eine Klebeschicht (6) und gegebenenfalls einen undurchlässigen Träger (2) umfasst, wobei die Wirkstoffreservoirschicht (3) in dem die Wirkstoffreservoirschicht bildenden Film eingeschlossen ist oder zwischen dem die Wirkstoffreservoirschicht bildenden Film (4) und dem Träger, der auf dem die Wirkstoffreservoirschicht bildenden Film (4) gebildet ist, eingeschlossen ist, wobei die Wirkstoffreservoirschicht (3) einen Wirkstoff außer 2-tert-Butylamino-1-(2-chlor-4-hydroxyphenyl)ethan-1-ol und einem pharmakologisch annehmbaren Salz davon enthält, **dadurch gekennzeichnet, dass** sich die Textilschicht (5) zwischen der Klebeschicht (6) und dem die Wirkstoffreservoirschicht bildenden Film (4) befindet.

2. Perkutan resorbierbares Präparat (1) gemäß Anspruch 1, wobei die Wirkstoffreservoirschicht (3) weiterhin einen Lösungsvermittler oder einen Promotor der perkutanen Resorption oder sowohl einen Lösungsvermittler als auch einen Promotor der perkutanen Resorption einschließt.

3. Perkutan resorbierbares Präparat (1) gemäß Anspruch 2, wobei der Lösungsvermittler ein Alkohol mit 1 bis 4 Kohlenstoffatomen ist.

4. Perkutan resorbierbares Präparat (1) gemäß Anspruch 2, wobei der Promotor der perkutanen Resorption wenigstens ein Vertreter ist, der aus der Gruppe ausgewählt ist, die aus einem Ester einer Fettsäure mit 12 bis 18 Kohlenstoffatomen, einem Diester einer Dicarbonsäure mit 6 bis 10 Kohlenstoffatomen und einem Glycerinester einer Fettsäure mit 8 bis 10 Kohlenstoffatomen besteht.

5. Perkutan resorbierbares Präparat gemäß Anspruch 1, wobei der die Wirkstoffreservoirschicht bildende Film (4) aus einem Ethylen-Vinylacetat-Copolymer mit einem Vinylacetatgehalt von 5-40 Gew.-% besteht.

6. Perkutan resorbierbares Präparat gemäß Anspruch 1, das weiterhin einen pH-Regulator in der Wirkstoffreservoirschicht (3) oder der Klebeschicht (6) oder sowohl der Wirkstoffreservoirschicht (3) als auch der Klebeschicht (6) umfasst.

## Revendications

1. Préparation absorbable par voie percutanée comprenant une couche réservoir de médicament (3), un film formant la couche réservoir de médicament (4), une couche de tissu (5), une couche adhésive (6) et de façon optionnelle un support imperméable (2), dans laquelle la couche réservoir de médicament (3) est enfermée dans le film formant la couche réservoir de médicament ou est enfermée par le film formant la couche réservoir de médicament (4) et le support formé sur le film formant la couche réservoir de médicament (4), la couche réservoir de médicament (3) contient un médicament exception faite du 2-tert-butylamino-1-(2-chloro-4-hydroxyphényl)éthan-1-ol et d'un sel pharmaceutiquement acceptable de celui-ci, **caractérisée en ce que** la couche de tissu (5) est placée entre la couche adhésive (6) et le film formant la couche réservoir de médicament (4).

2. Préparation absorbable par voie percutanée (1) selon la revendication 1, dans laquelle la couche réservoir de médicament (3) renferme en outre un solubilisant ou un agent favorisant l'absorption percutanée, ou un solubilisant et un agent favorisant l'absorption percutanée.

3. Préparation absorbable par voie percutanée (1) selon la revendication 2, dans laquelle le solubilisant est un alcool ayant 1 à 4 atomes de carbone.

4. Préparation absorbable par voie percutanée (1) selon la revendication 2, dans laquelle l'agent favorisant l'absorption percutanée est au moins un élément choisi dans le groupe constitué par un ester d'acide gras ayant 12 à 18 atomes de carbone, un diester d'acide dicarboxylique ayant 6 à 10 atomes de carbone et un ester de glycérol d'un acide gras ayant 8 à 10 atomes de carbone.

5. Préparation absorbable par voie percutanée selon la revendication 1, dans laquelle le film formant la couche réservoir de médicament (4) est réalisé en copolymère éthylène - acétate de vinyle ayant un taux d'acétate de vinyle de 5 à 40 % en masse.

6. Préparation absorbable par voie percutanée selon la revendication 1, comprenant en outre un agent ajustant le pH dans la couche réservoir de médicament (3) ou dans la couche adhésive (6), ou dans la couche réservoir de médicament (3) et dans la couche adhésive (6).
